# EUROPEAN PATENT APPLICATION

(11) **EP 1 535 920 A1**
(43) Date of publication of application: **01.06.2005**
(21) Application number: 03762875.7
(22) Date of filing: 03.07.2003
(51) Int. Cl.: C07D 317/72

(54) **PROCESS FOR PREPARATION OF 1,3-BENZODIOXOLE-2-SPIRO- CYCLOALKANE DERIVATIVES**

(30) Priority: 03.07.2002 JP 2002194273
(71) Applicant: KYOWA HAKKO KOGYO CO., LTD., Chiyoda-ku, Tokyo 100-8185 (JP)
(72) Inventor: ATSUMI, Toshiyuki, Sakai Research Laboratories, Sakai-shi, Osaka 590-8554 (JP); YANAGISAWA, Arata, Sakai Research Laboratories, Sakai-shi, Osaka 590-8554 (JP); CHUJO, Iwao, Sakai Research Laboratories, Sakai-shi, Osaka 590-8554 (JP); TSUMUKI, Hiroshi, Kyowa Hakko Kogyo Co., Ltd., Tokyo 100-8185 (JP); MOHRI, Shin-ichiro, Sakai Plant, Sakai-shi, Osaka 590-8554 (JP)
(74) Representative: Casalonga, Axel
(86) International application number: PCT/JP2003/008478
(87) International publication number: WO 2004/005276

(57) **Abstract**

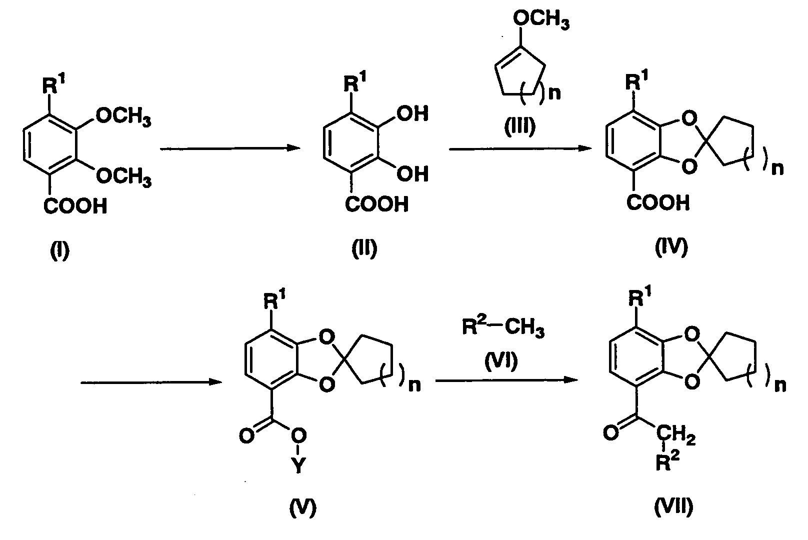

(wherein R¹ represents hydroxy or substituted or unsubstituted lower alkoxy; R² represents an aromatic heterocyclic group or the like; Y represents lower alkyl or the like; and n represents an integer of from 1 to 6)

For example, a process for preparing a 1,3-benzodioxole-2-spirocycloalkane derivative represented by the above formula (VII), which comprises adding a base to a mixture containing a compound represented by the above formula (V) and a compound represented by the above formula (VI); and allowing the compound represented by the above formula (V) to react with the compound represented by the above formula (VI), are provided.

## Description

### Technical Field

The present invention relates to a process for preparing a 1,3-benzodioxole-2-spirocycloalkane derivative having inhibitory activity of phosphodiesterase (PDE) IV which is useful as therapeutic agents of, for example, an inflammatory or an allergic disease such as bronchial asthma, allergic rhinitis, nephritis or the like; an autoimmune disease such as rheumatism, multiple sclerosis, Crohn's disease, psoriasis, systemic lupus erythematosus or the like; a disease of central nervous system such as depression, amnesia, dementia or the like; an organ disease with ischemia-reperfusion injury caused by, for example, cardiac failure, shock, cerebral ischemia injury or the like; insulin-resistant diabetes, wounds, acquired immune deficiency syndrome (AIDS) or the like.

### Background Art

WO96/36624 discloses that compounds containing 1,3-benzodioxole-2-spirocycloalkane derivatives represented by formula (VII) 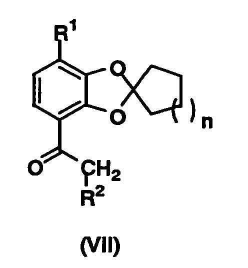 (wherein R¹ represents hydroxy, or substituted or unsubstituted lower alkoxy; R² represents substituted or unsubstituted aryl, or a substituted or unsubstituted aromatic heterocyclic group; and n represents an integer of from 1 to 6)
have inhibitory activity of PDE IV and are useful as therapeutic agents of, for example, asthma, allergy, rheumatism, psoriasis, myocardial infarct, depression, amnesia, multiple sclerosis, Crohn's disease, systemic lupus erythematosus, diabetes, wounds, AIDS or the like. A process for preparing the 1,3-benzodioxole-2-spirocycloalkane derivative disclosed in WO96/36624 is as follows.

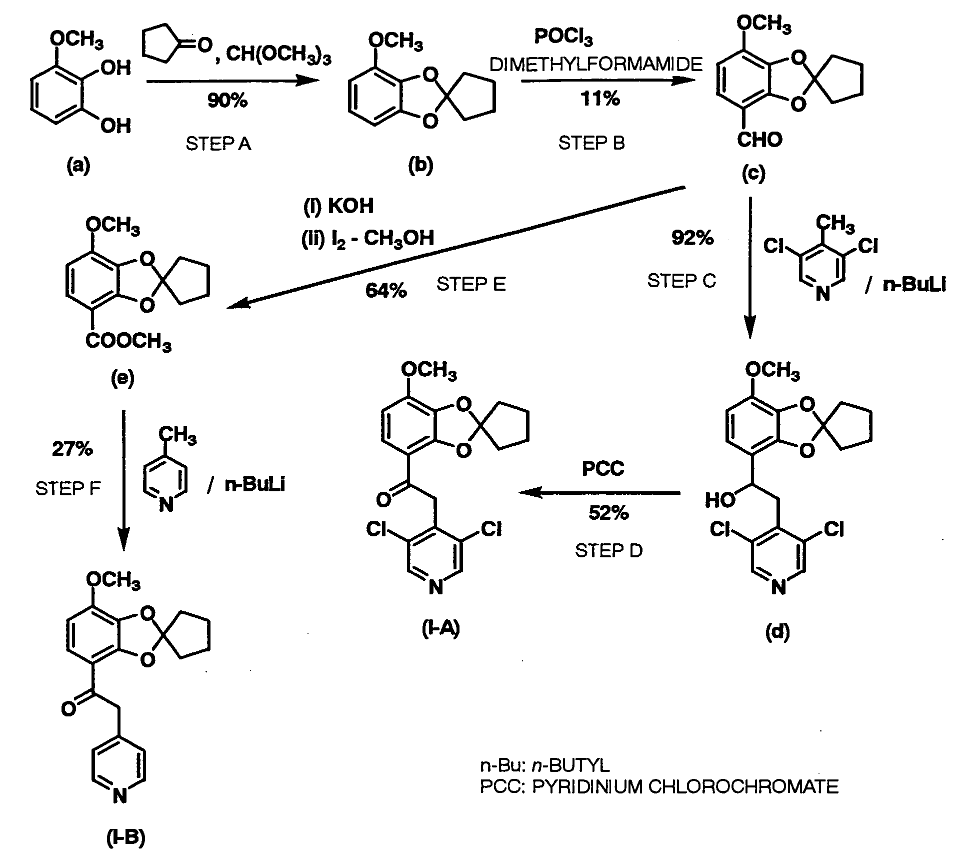

However, the process disclosed in WO96/36624 has, for example, the following problems: (1) Since Compound (b) prepared in step A is oily, isolating and purifying Compound (b) by crystallization are difficult. (2) In steps B, C, D and F, silica-gel column chromatography is required for purification. (3) The total yield is low, i.e., around 5% because of a low yield in each step. To provide a large amount of the desired 1,3-benzodioxole-2-spirocycloalkane derivative and intermediates for the manufacture thereof, the above-described problems should be solved. That is, it is required to develop a simple and efficient industrial process for preparation having a high yield and avoiding complicated purification steps such as silica-gel column chromatography or the like.

### Disclosure of the Invention

An object of the present invention is to provide a simple process with a high yield for mass-producing a 1,3-benzodioxole-2-spirocycloalkane derivative which has inhibitory activity of PDE IV and is useful as therapeutic agents of, for example, an inflammatory or allergic disease such as bronchial asthma, allergic rhinitis, nephritis or the like; an autoimmune disease such as rheumatism, multiple sclerosis, Crohn's disease, psoriasis, systemic lupus erythematosus or the like; a disease of central nervous system such as depression, amnesia, dementia or the like; an organ disease with ischemia-reperfusion injury caused by, for example, cardiac failure, shock, cerebral ischemia injury or the like; insulin-resistant diabetes, wounds, AIDS or the like.

The present invention relates to the following (1) to (11).
(1) A process for preparing a 1,3-benzodioxole-2-spirocycloalkane derivative represented by formula (VII) 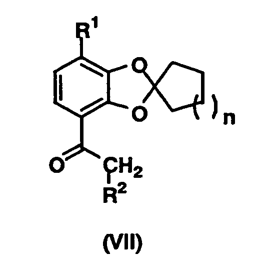 (wherein R¹ represents hydroxy, or substituted or unsubstituted lower alkoxy; R² represents substituted or unsubstituted aryl, or a substituted or unsubstituted aromatic heterocyclic; and n represents an integer of from 1 to 6),
   which comprises treating a compound represented by formula (I) 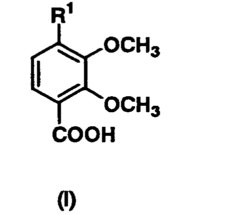 (wherein R¹ has the same meaning as defined above) with hydrogen iodide to give a compound represented by formula (II) 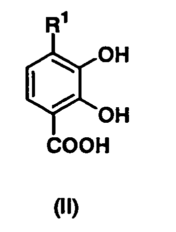 (wherein R¹ has the same meaning as defined above);
   allowing the resulting compound represented by the above formula (II) to react with a compound represented by formula (III) 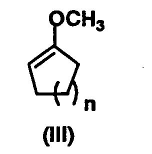 (wherein n has the same meaning as defined above)
   to give a compound represented by formula (IV) 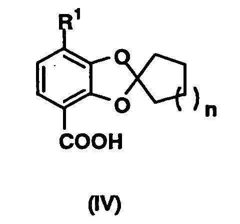 (wherein R¹ and n have the same meanings as defined above respectively);
   converting the resulting compound represented by the above formula (IV) into a compound represented by formula (V) 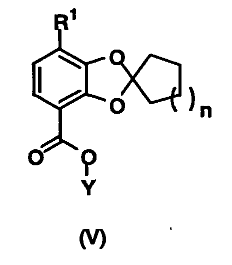 (wherein R¹ and n have the same meanings as defined above respectively, and Y represents lower alkyl, lower alkenyl, lower alkynyl, substituted or unsubstituted aralkyl, substituted or unsubstituted aryl, or a substituted or unsubstituted aromatic heterocyclic group);
   adding a base to a mixture containing the resulting compound represented by the above formula (V) and a compound represented by formula (VI)

   **R**^{**2**}**-CH**_{**3**} **(VI)**

   (wherein R² has the same meaning as defined above);
   and allowing the compound represented by the above formula (V) to react with the compound represented by the above formula (VI).
(2) A process for preparing a 1,3-benzodioxole-2-spirocycloalkane derivative represented by formula (VII) 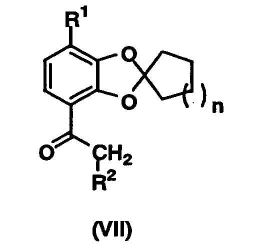 (wherein R¹, R² and n have the same meanings as defined above respectively),
   which comprises adding a base to a mixture containing a compound represented by formula (V) 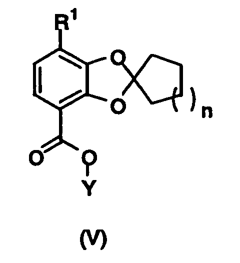 (wherein R¹, n and Y have the same meanings as defined above, respectively)
   and a compound represented by formula (VI)

   **R**^{**2**}**-CH**_{**3**} **(VI)**

   (wherein R² has the same meaning as defined above);
   and allowing the compound represented by the above formula (V) to react with the compound represented by the above formula (VI).
(3) The process for preparing a 1,3-benzodioxole-2-spirocycloalkane derivative according to above (1) or (2), wherein the base is lithium bis(trimethylsilyl)amide.
(4) The process for preparing a 1,3-benzodioxole-2-spirocycloalkane derivative according to above (3), wherein the reaction temperature when the compound represented by formula (V) reacts with the compound represented by formula (VI) is between -10° C and 50° C.
(5) The process for preparing a 1,3-benzodioxole-2-spirocycloalkane derivative according to any one of above (1) to (4), wherein Y is *n*-butyl.
(6) A process for preparing a 1,3-benzodioxole-2-spirocycloalkane derivative represented by formula (VII) 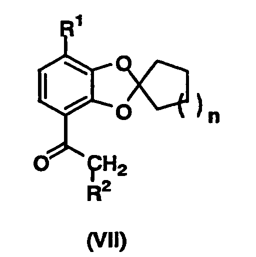 (wherein R¹, R² and n have the same meanings as defined above respectively),
   which comprises allowing a compound represented by formula (II) 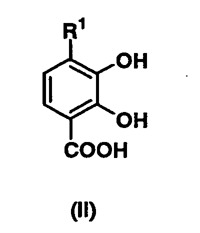 (wherein R¹ has the same meaning as defined above)
   to react with a compound represented by formula (III) 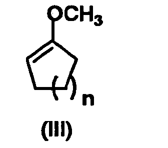 (wherein n represents an integer of from 1 to 6),
   to give a compound represented by formula (IV) 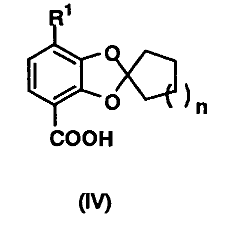 (wherein R¹ and n have the same meanings as defined above respectively);
   converting the resulting compound represented by the above formula (IV) into a compound represented by formula (V) 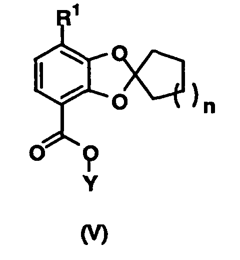 (wherein R¹, n, and Y have the same meanings as defined above respectively);
   adding a base to a mixture containing the resulting compound represented by the above formula (V) and a compound represented by formula (VI)

   **R**^{**2**}**-CH**_{**3**} **(VI)**

   (wherein R² has the same meaning as defined above);
   and allowing the compound represented by the above formula (V) to react with the compound represented by the above formula (VI).
(7) A process for preparing a compound represented by formula (IV) 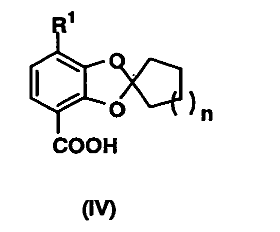 (wherein R¹ and n have the same meanings as defined above respectively),
   which comprises allowing a compound represented by formula (II) 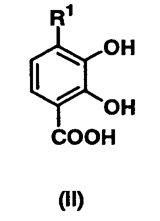 (wherein R¹ has the same meaning as defined above)
   to react with a compound represented by formula (III) 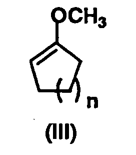 (wherein n has the same meaning as defined above).
(8) A process for preparing a 1,3-benzodioxole-2-spirocycloalkane derivative represented by formula (VII) 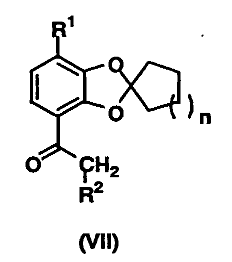 (wherein R¹, R² and n have the same meanings as defined above respectively),
   which comprises converting a compound represented by formula (IV) 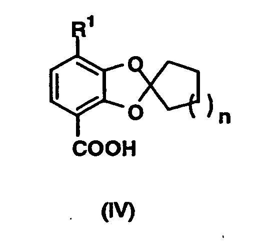 (wherein R¹ and n have the same meanings as defined above respectively)
   into a compound represented by formula (V) 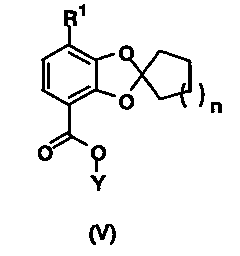 (wherein R¹, n and Y have the same meanings as defined above respectively);
   adding a base to a mixture containing the resulting compound represented by the above formula (V) and a compound represented by formula (VI)

   **R**^{**2**}**-CH**_{**3**} **(VI)**

   (wherein R² has the same meaning as defined above);
   and allowing the compound represented by the above formula (V) to react with the compound represented by the above formula (VI).
(9) A process for preparing a compound represented by formula (II) 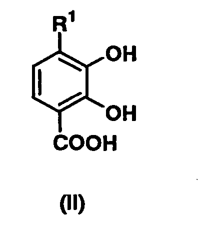 (wherein R¹ has the same meaning as defined above),
   which comprises treating a compound represented by formula (I) 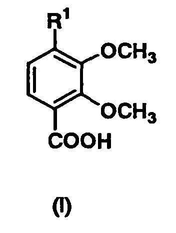 (wherein R¹ has the same meaning as defined above) with hydrogen iodide.
(10) The process for preparing according to any one of above (1) to (6) or (8), wherein R² is a substituted or unsubstituted aromatic heterocyclic group.
(11) The process for preparing according to any one of above (1) to (10), wherein R¹ is methoxy.

Hereinafter, the compound represented by formula (I) is referred to as "Compound (I)". The term "Compound (I-A)" means that "Compound (I-A)" is included in Compound (I). Compounds represented by other formula number are similarly referred to.

In the definition of each group in compounds (I) to (VII), examples of the lower alkyl and a alkyl moiety of the lower alkoxy include, for example, straight or branched alkyl having 1 to 10 carbon atoms, such as methyl, ethyl, propyl, isopropyl, butyl, isobutyl, *sec*-butyl, *tert*-butyl, pentyl, neopentyl, hexyl, heptyl, octyl, nonyl, decyl and the like.

Examples of the lower alkenyl include, for example, straight or branched alkenyl having 2 to 8 carbon atoms, such as vinyl, allyl, 1-propenyl, butenyl, pentenyl, hexenyl, heptenyl, octenyl and the like.

Examples of the lower alkynyl include, for example, straight or branched alkynyl having 2 to 8 carbon atoms, such as ethynyl, propynyl, butynyl, pentynyl, hexynyl, heptynyl, octynyl and the like.

Examples of the aralkyl include, for example, aralkyl having 7 to 20 carbon atoms, such as benzyl, naphthylmethyl, triphenylmethyl and the like.

Examples of the aryl include, for example, aryl having 6 to 14 carbon atoms, such as phenyl, naphthyl, indenyl, anthryl and the like.

Examples of the aromatic heterocyclic group include, for example, 5- or 6-membered monocyclic aromatic heterocyclic groups containing at least one atom selected from a nitrogen atom, an oxygen atom and a sulfur atom; and bicyclic or tricyclic condensed aromatic heterocyclic groups comprising 3- to 8-membered rings and containing at least one atom selected from a nitrogen atom, an oxygen atom and a sulfur atom, and the like, such as furyl, thienyl, pyrrolyl, pyridyl, oxazolyl, thiazolyl, imidazolyl, triazolyl, pyrimidinyl, pyrazinyl, pyridazinyl, triazinyl, indolyl, quinolyl, cinnolinyl, quinazolinyl, quinoxalinyl, phthalazinyl, purinyl, benzoxazolyl, benzothiazolyl, benzoimidazolyl, benzotriazolyl and the like.

Examples of substituents in the substituted lower alkoxy, the substituted aralkyl, the substituted aryl, and the substituted aromatic heterocyclic group are same or different and include, for example, 1 to 3 substituent(s), such as lower alkyl, lower alkoxy, lower alkylthio, lower alkylamino, hydroxy, amino, halogen, nitro, cyano and the like.

Herein, lower alkyl and lower alkyl moiety of the lower alkoxy, lower alkylthio and lower alkylamino described above have the same meanings as the lower alkyl described above. Halogen means each atom of fluorine, chlorine, bromine and iodine.

Embodiments of a process for preparing Compound (VII) according to the present invention will be described below.

In the process described below, when the defined functional groups are changed under the conditions of the method performed or are inappropriate to perform the method, common methods used in organic synthesis such as protection and deprotection of functional groups [see, Protective Groups in Organic Synthesis. Wiley-Interscience Publication, page 309 (1991)], oxidation, reduction, hydrolysis and the like, are employed. As a result, preparations can be easily performed.

Compound (VII) may be prepared by the following process.

### PROCESS 1

Compound (VII) can be prepared by the following series of steps starting from Compound (II): 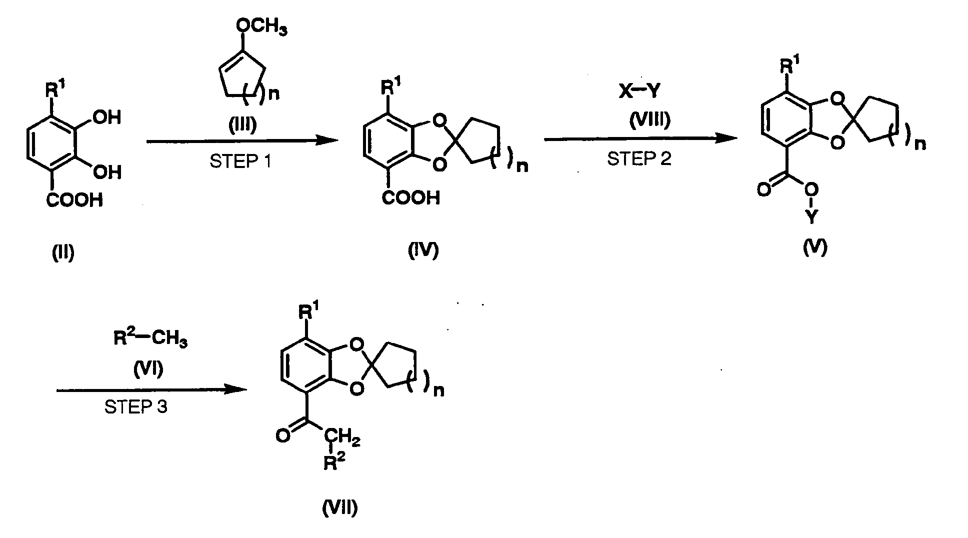 (Wherein R¹, R², Y and n have the same meanings as defined above respectively; X represents halogen, lower alkylsulfonyloxy, or substituted or unsubstituted arylsulfonyloxy, herein the halogen, the alkyl moiety of the lower alkylsulfonyloxy and the aryl moiety of the arylsulfonyloxy have the same meanings as the above-described halogen, lower alkyl and aryl, respectively; and the substituents in the substituted arylsulfonyloxy has the same meaning as the substituents in the above-described substituted aryl.)

Compound (II) may be obtained as commercially available product, or prepared by known methods [for example, see Synthetic Communications, Vol. 16, page 645 (1986) and the like] or similar methods thereto. Alternatively, according to PROCESS 2 described below, Compound (II) can be prepared more efficiently.

Compound (III) may be obtained as commercially available product, or prepared by known methods [for example, see Synthesis, page 38 (1974) and the like] or similar methods thereto.

Compound (VIII) may be obtained as commercially available product, or prepared by known methods [for example, see Shinjikkenkagakukoza, Synthesis and reaction of organic compound (III), Vol. 14, page 1793 (1978) and the like] or similar methods thereto.

Compound (VI) may be obtained as commercially available product, or prepared by known methods [for example, see WO94/20455, Tetrahedron Lett. Vol. 37, page 2565 (1996) and the like] or similar methods thereto.

### STEP 1: PREPARATION OF COMPOUND (IV)

Compound (II) reacts with 1 equivalent to a large excess of, preferably 10 to 200 equivalents of Compound (III) in an inert solvent or without a solvent usually at a temperature between 10° C and 150°C, preferably between 100°C and 150°C for 10 minutes to 48 hours, to give Compound (IV).

Examples of the inert solvents include, for example, aromatic hydrocarbons such as benzene, toluene, xylene and the like; aliphatic hydrocarbons such as pentane, hexane, cyclohexane and the like; dichloromethane, dichloroethane, chloroform, carbon tetrachloride, trifluorotoluene, dichlorobenzene, chlorobenzene, tetralin, diphenyl ether, dioxane, dimethoxyethane, ethylene glycol dimethyl ether, diethyl ether, diisopropyl ether, tetrahydrofuran, cyclopentanone, cyclohexanone, methyl ethyl ketone, sulfolane, ethyl acetate, propyl acetate, butyl acetate and the like, but are not limited thereto so long as they are inert to the reaction. Among them, cyclopentanone, cyclohexanone and the like are preferable, and these may be used alone or in combination.

### STEP 2: PREPARATION OF COMPOUND (V)

Compound (IV) prepared in step 1 reacts with 1 to 10 equivalents of, preferably 1 to 1.5 equivalents of Compound (VIII) in an inert solvent or without a solvent in the presence of 1 to 10 equivalents of a base usually at a temperature between -10° C and 150° C, preferably between 10° C and 70°C for 10 minutes to 48 hours, to give Compound (V).

Examples of the bases include, for example, organic bases such as triethylamine, tributylamine, diisopropylethylamine, pyridine, 4-dimethylaminopyridine, 1,8-diazabicyclo[5.4.0]undecene, 1,4-diazabicyclo[2.2.2]octane, N-methylmorpholine and the like; inorganic bases such as sodium hydroxide, potassium hydroxide, lithium hydroxide, calcium hydroxide, magnesium hydroxide, cesium hydroxide, sodium carbonate, potassium carbonate, cesium carbonate, calcium carbonate, magnesium carbonate, sodium hydrogen carbonate, potassium hydrogen carbonate, sodium phosphate, potassium phosphate, sodium acetate, potassium acetate, potassium *tert*-butoxide, sodium *tert*-butoxide, sodium methoxide, sodium ethoxide, sodium hydride, potassium hydride, calcium hydride and the like; and the like. Among them, sodium carbonate, potassium carbonate and the like are preferable.

Examples of the inert solvents include, for example, aliphatic hydrocarbons such as pentane, hexane, cyclohexane and the like; aromatic hydrocarbons such as benzene, toluene, xylene and the like; tetralin, diphenyl ether, ethyl acetate, dichloromethane, chloroform, dichloroethane, carbon tetrachloride, trifluorotoluene, dichlorobenzene, chlorobenzene, pyridine, ethyl acetate, propyl acetate, butyl acetate, acetone, acetonitrile, N,N-dimethylformamide, N,N-dimethylacetamide, 1-methyl-2-pyrrolidone, 1,3-dimethyl-2-imidazolidinone, dimethyl sulfoxide, sulfolane, dimethyl sulfone, tetrahydrofuran, dioxane, dimethoxyethane, ethylene glycol dimethyl ether, diethyl ether, diisopropyl ether, methanol, ethanol, propanol, butanol, water and the like, but are not limited thereto so long as they are inert to the reaction. Among them, N,N-dimethylformamide, N,N-dimethylacetoamide, 1-methyl-2-pyrrolidone, 1,3-dimethyl-2-imidazolidinone and the like are preferable, and these may be used alone or in combination.

Compound (V) prepared in this step can be used for the next step. To obtain Compound (V) as a crystal, the Compound (V) of which Y is n-butyl is preferable.

### STEP 3: PREPARATION OF COMPOUND (VII)

Compound (V) prepared in step 2 and 1 to 10 equivalents of, preferably 1 to 2 equivalents of Compound (VI) are dissolved in an inert solvent. Then, to the resulting solution is added 1 to 5 equivalents, preferably 2 to 4 equivalents of a base usually at a temperature between -78° C and 50°C, preferably between -10°C and 50°C, and the mixture is reacted at the same temperature for 5 minutes to 24 hours, to give Compound (VII).

Examples of the inert solvent include, for example, aliphatic hydrocarbons such as pentane, hexane, cyclohexane and the like; aromatic hydrocarbons such as benzene, toluene, xylene and the like; tetralin, trifluorotoluene, chlorobenzene, diphenyl ether, pyridine, acetonirile, N,N-dimethylformamide, N,N-dimethylacetoamide, 1-methyl-2-pyrrolidone, 1,3-dimethyl-2-imidazolidinone, dimethyl sulfoxide, diethyl ether, diisopropyl ether, tetrahydrofuran, dioxane, dimethoxyethane, ethylene glycol dimethyl ether and the like, but are not limited thereto so long as they are inert to the reaction. Among them, diethyl ether, tetrahydrofuran, hexane and the like are preferable, and these may be used alone or in combination.

Examples of the bases include, for example, *n*-butyllithium, *sec*-butyllithium, *tert*-butyllithium, lithium diethylamide, lithium diisopropylamide, lithium cyclohexylamide, lithium bis(trimethylsilyl)amide, sodium bis(trimethylsilyl)amide, potassium bis(trimethylsilyl)amide, methylmagnesium bromide, ethylmagnesium bromide, propylmagnesium bromide, isopropylmagnesium bromide, phenylmagnesium bromide, ethylmagnesium chloride, propylmagnesium chloride, isopropylmagnesium chloride, sodium hydride, potassium hydride, potassium *tert*-butoxide and the like. Among them, lithium diisopropylamide lithium bis(trimethylsilyl)amide and the like are preferable.

### PROCESS 2

Compound (II) may be obtained as commercially available product, or prepared by known methods [for example, see Synthetic Communications, vol.16, page 645 (1986) and the like] or similar methods thereto. Alternatively, according to the following method, Compound (II) can be prepared more efficiently. 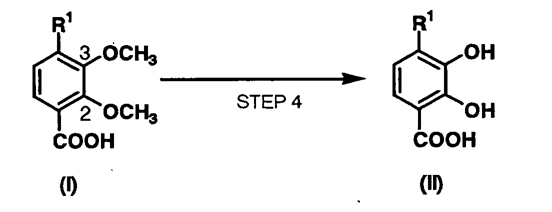 ( Wherein R¹ has the same meaning as defined above.)

### STEP 4: PREPARATION OF COMPOUND (II)

Compound (I) is treated with 2 to 10 equivalents of hydrogen iodide in a solvent usually at a temperature between 0°C and 100°C for 5 minutes to 48 hours to give Compound (II).

Examples of the solvents include, but not limited to, for example, water, formic acid, acetic acid, propionic acid, butyric acid, trifluoroacetic acid, chloroacetic acid, dichloroacetic acid, trichloroacetic acid, acetonitrile, propionitrile, methanol, ethanol, propanol, butanol, isopropyl alcohol, ethylene glycol, glycerin, ethylene glycol monomethyl ether, dimethyl sulfoxide, sulfolane, N,N-dimethylformamide, N,N-dimethylacetamide, 1-methyl-2-pyrrolidone, 1,3-dimethyl-2-imidazolidinone, dioxane, ethyl acetate, propyl acetate, butyl acetate, pentane, hexane, cyclohexane, benzene, toluene, xylene, tetralin, trifluorotoluene, chlorobenzene, diphenyl ether and the like. Among them, water, acetic acid and the like are preferable, and these may be used alone or in combination.

Compound (I) may be obtained as commercially available product.

According to this process, the two methoxy groups at the 2-position and the 3-position of Compound (I) can be efficiently converted into hydroxy groups with high selectivity.

### PROCESS 3

Compound (V) can also be prepared by the following process. 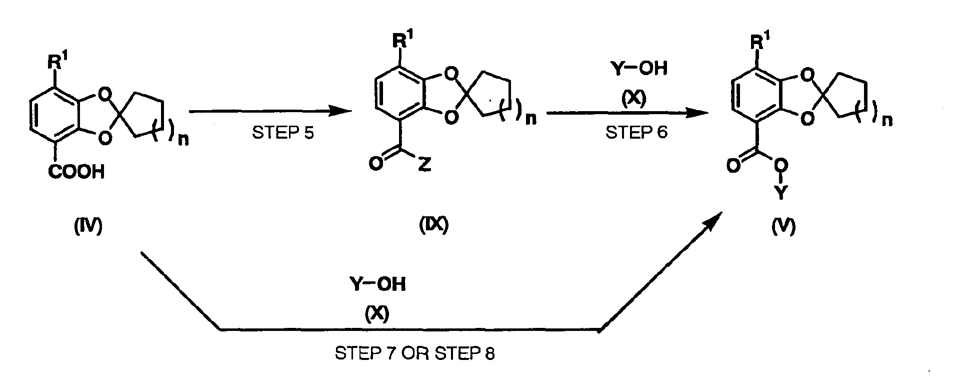 (Wherein R¹, Y and n have the same meanings as defined above respectively; Z represents halogen which has the same meaning as the above-described halogen.)

### STEP 5: PREPARATION OF COMPOUND (IX)

Compound (IV) prepared in step 1 of PROCESS 1 is treated with 1 equivalent to a large excess of, preferably 1 to 10 equivalents of a halogenating agent in an inert solvent or without a solvent usually at a temperature between -10°C and 100°C for 10 minutes to 48 hours, to give Compound (IX).

Examples of the halogenating agents include, for example, thionyl chloride, phosphorus oxychloride, phosphorus oxybromide, oxalyl dichloride, phosgene and the like.

Examples of the inert solvents include, for example, aliphatic hydrocarbons such as pentane, hexane, cyclohexane and the like; aromatic hydrocarbons such as benzene, toluene, xylene and the like; dichloromethane, dichloroethane, chloroform, carbon tetrachloride, trifluorotoluene, dichlorobenzene, chlorobenzene, tetralin, diphenyl ether, pyridine, acetonitrile, N,N-dimethylformamide, N,N-dimethylacetoamide, 1-methyl-2-pyrrolidone, 1,3-dimethyl-2-imidazolidinone, diethyl ether, diisopropyl ether, tetrahydrofuran, dioxane, dimethoxyethane, ethylene glycol dimethyl ether, methyl ethyl ketone, acetone, ethyl acetate, propyl acetate, butyl acetate and the like, but are not limited thereto so long as they are inert to the reaction. These may be used alone or in combination.

Furthermore, if necessary, a catalytic amount to 10 equivalents of, preferably 0.1 to 5 equivalents of a base, such as pyridine, triethylamine and the like, and/or N,N-dimethylformamide may be added.

### STEP 6: PREPARATION OF COMPOUND (V)

Compound (IX) prepared in step 5 reacts with 1 equivalent to a large excess of, preferably 1 to 200 equivalents of Compound (X) in an inert solvent or without a solvent in the presence or absence of a base usually at a temperature between -10°C and 100°C for 10 minutes to 48 hours, to give Compound (V).

Examples of the inert solvents include, for example, aliphatic hydrocarbons such as pentane, hexane, cyclohexane and the like; aromatic hydrocarbons such as benzene, toluene, xylene and the like; dichloromethane, dichloroethane, chloroform, carbon tetrachloride, trifluorotoluene, dichlorobenzene, chlorobenzene, tetralin, diphenyl ether, pyridine, acetonitrile, N,N-dimethylformamide, N,N-dimethylacetoamide, 1-methyl-2-pyrrolidone, 1,3-dimethyl-2-imidazolidinone, dimethyl sulfoxide, diethyl ether, diisopropyl ether, tetrahydrofuran, dioxane, dimethoxyethane, ethylene glycol dimethyl ether, methyl ethyl ketone, acetone, ethyl acetate, propyl acetate, butyl acetate and the like, but are not limited thereto so long as they are inert to the reaction. These may be used alone or in combination.

Examples of the bases include, for example, organic bases such as pyridine, 4-dimethylaminopyridine, N,N-diethylaniline, triethylamine, diisopropylethylamine, 1,8-diazabicyclo[5.4.0]undecene, 1,4- diazabicyclo[2.2.2]octane, N-methylmorpholine and the like; and inorganic bases such as sodium hydroxide, potassium hydroxide, lithium hydroxide, calcium hydroxide, magnesium hydroxide, cesium hydroxide, sodium carbonate, potassium carbonate, cesium carbonate, calcium carbonate, magnesium carbonate, sodium hydrogen carbonate, potassium hydrogen carbonate, sodium phosphate, potassium phosphate, sodium acetate, potassium acetate, potassium *tert*-butoxide, sodium *text*-butoxide, sodium methoxide, sodium ethoxide, sodium hydride, potassium hydride, calcium hydride and the like.

In this step, Compound (IX) which is prepared in step 5 may also be used without isolation in Step 5.

### STEP 7: PREPARATION OF COMPOUND (V)

Compound (IV) reacts with 1 equivalent to a large excess of, preferably 1 to 200 equivalents of Compound (X) in the presence of 1 to 10 equivalents of a halogenating agent usually at a temperature between -10°C and 100°C for 10 minutes to 48 hours in an inert solvent or without a solvent, to give Compound (V).

Examples of the halogenating agents include, for example, thionyl chloride, phosphorus oxychloride, phosphorus oxybromide, oxalyl dichloride, phosgene and the like.

Examples of the inert solvents include, for example, aliphatic hydrocarbons such as pentane, hexane, cyclohexane and the like; aromatic hydrocarbons such as benzene, toluene, xylene and the like; dichloromethane, dichloroethane, chloroform, carbon tetrachloride, trifluorotoluene, dichlorobenzene, chlorobenzene, tetralin, diphenyl ether, pyridine, acetonitrile, N,N-dimethylformamide, N,N-dimethylacetoamide, 1-methyl-2-pyrrolidone, 1,3-dimethyl-2-imidazolidinone, diethyl ether, diisopropyl ether, tetrahydrofuran, dioxane, dimethoxyethane, ethylene glycol dimethyl ether, methyl ethyl ketone, acetone, ethyl acetate, propyl acetate, butyl acetate and the like, but are not limited thereto so long as they are inert to the reaction. These may be used alone or in combination.

### STEP 8: PREPARATION OF COMPOUND (V)

Compound (IV) reacts with 1 equivalent to a large excess of, preferably 1 to 10 equivalents of Compound (X) in an inert solvent or without a solvent in the presence of a catalytic amount to 10 equivalents of, preferably 0.1 to 5 equivalents of a condensing reagent usually at a temperature between -10°C and 100°C for 10 minutes to 48 hours, to give Compound (V).

Furthermore, if necessary, an additive such as 1-hydroxybenzotriazole and the like may be added.

Examples of the inert solvents include, for example, aliphatic hydrocarbons such as pentane, hexane, cyclohexane and the like; aromatic hydrocarbons such as benzene, toluene, xylene and the like; dichloromethane, dichloroethane, chloroform, carbon tetrachloride, trifluorotoluene, dichlorobenzene, chlorobenzene, tetralin, diphenyl ether, pyridine, acetonitrile, N,N-dimethylformamide, N,N-dimethylacetoamide, 1-methyl-2-pyrrolidone, 1,3-dimethyl-2-imidazolidinone, diethyl ether, diisopropyl ether, tetrahydrofuran, dioxane, dimethoxyethane, ethylene glycol dimethyl ether, methyl ethyl ketone, acetone, ethyl acetate, propyl acetate, butyl acetate and the like, but are not limited thereto so long as they are inert to the reaction. These may be used alone or in combination.

Examples of the condensing reagents include condensing reagents used for peptide synthesis, and the like, for example, N,N'-dicyclohexylcarbodiimide 1-ethyl-3-(3-dimethylaminopropyl)carbodiimide and the like.

The intermediates and the desired compound prepared in the above-described process may be isolated and purified by applying to any purification methods which are commonly used in organic synthetic chemistry such as filtration, extraction, washing, condensation, recrystallization, various chromatographies and the like. These intermediates may be used for the subsequent reaction without particularly purification.

The present invention will now be described in detail with examples but the present invention is not limited thereto.

### Best Mode for Carrying Out the Invention

### EXAMPLE 1: 2-(3,5-dichloropyridine-4-yl)-1-[(7-methoxy-1,3-benzodioxole-2-spirocyclopentane)-4-yl]ethan-1-one (Compound (VII-A))

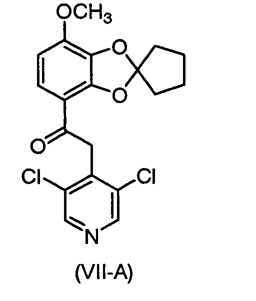

### STEP 1: 2,3-dihydroxy-4-methoxybenzoic acid

2,3,4-Trimethoxybenzoic acid (300.0 g, 1.41 mol) was dissolved in acetic acid (1.8 L), and to the solution was added 55% hydriodic acid (750 ml) with stirring at room temperature, and then heated to 80°C. The reaction mixture was stirred at 80°C for 10 hours. After confirming the disappearance of 2,3,4-trimethoxybenzoic acid by high-performance liquid chromatography (HPLC), the reaction mixture was cooled to room temperature, and then the pH of the mixture was adjusted to 1.5 by the dropwise addition of a 5 mol/L aqueous sodium hydroxide (600 ml). After being further stirring at room temperature for 1 hour, the precipitated solid was filtered, and the resulting solid was washed with water (1.8 L), followed by drying at 50°C for 5 hours under reduced pressure to give 2,3-dihydroxy-4-methoxybenzoic acid(191 g, 73% yield) as a white solid. Melting point: 234° C
¹H NMR (300 MHz, DMSO-d₆) δ (ppm): 8.54 (brs, 1H), 7.29 (d, J=9.9 Hz, 1H), 6.58 (d, J=9.9 Hz, 1H), 3.81 (s, 3H)
EI-MS: 184 (M⁺)
IR (KBr, cm⁻¹): 1651, 1508, 1439, 1394, 1281, 1088, 899, 772

### STEP 2: (7-methoxy-1,3-benzodioxole-2-spirocyclopentane)-4-carboxylic acid

A mixture of 2,3-dihydroxy-4-methoxybenzoic acid (138 g, 450 mmol) prepared in step 1, 1-methoxycyclopentene (926 g, 9.4 mol) and cyclopentanone (828 ml) was stirred at 120°C for 6 hours. After confirming the disappearance of 2,3-dihydroxy-4-methoxybenzoic acid by HPLC, the reaction mixture was cooled to 45°C, and then the solvent was evaporated at 40° C to 50° C under reduced pressure of 10 mmHg to 50 mmHg. To the resulting residue was added toluene (1.6 L), and then the mixture was heated to 80°C with stirring. Next, the mixture was cooled to 3°C over a period of 2 hours with stirring, and the precipitated solid was filtered. The resulting solid was washed with toluene (414 ml), followed by drying at 50°C for 6 hours to give (7-methoxy-1,3-benzodioxole-2-spirocyclopentane)-4-carboxylic acid (166 g, 89% yield) as a white solid.
Melting point: 215°C
¹H NMR (300 MHz, CHCl₃) δ (ppm): 7.47 (d, J = 9.1 Hz, 1H), 6.56 (d, J=9.1 Hz, 1H), 3.96 (s, 3H), 2.26-2.16 (m, 4H), 1.92-1.86 (m, 4H)
EI-MS: 250 (M⁺)
IR (KBr, cm⁻¹): 1678, 1639, 1452, 1286, 1215, 1111, 766

### STEP 3: n-butyl (7-methoxy-1,3-benzodioxole-2-spirocyclopentane)-4-carboxylate

(7-methoxy-1,3-benzodioxole-2-spirocyclopentane)-4-carboxylic acid (50 g, 200 mmol) prepared in step 2 was dissolved in N,N-dimethylformamide (500 ml). To the solution were added potassium carbonate (27.6 g, 200 mmol) and subsequently *n*-butyl iodide (25 ml, 220 mmol), and the mixture was stirred at 50°C for 4 hours. After confirming the disappearance of (7-methoxy-1,3-benzodioxole-2-spirocyclopentane)-4-carboxylic acid by HPLC, the reaction mixture was cooled to room temperature. To the mixture was added dropwise water (500 ml), and the mixture was stirred for 1 hour under ice cooling. The resulting precipitated solid was filtered and washed with 500 ml of water, followed by drying at 25°C for 6 hours under reduced pressure to give n-butyl (7-methoxy-1,3-benzodioxole-2-spirocyclopentane)-4-carboxylate (59 g, 96% yield) as a white solid.
Melting point: 51° C
¹H NMR (300 MHz, CDCl₃) δ (ppm): 7.39 (d, J=9.1 Hz, 1H), 6.51 (d, J=9.1 Hz, 1H), 4.29 (t, J=6.5 Hz, 2H), 3.93 (s, 3H), 2.25-2.10 (m, 4H), 1.92-1.81 (m, 4H), 1.79-1.66 (m, 2H), 1.50-1.41 (m, 2H), 0.97 (t, J=7.4 Hz, 3H)
EI-MS: 306 (M⁺)
IR (KBr, cm⁻¹): 2955, 1701, 1639, 1450, 1285, 1215, 1107

### STEP 4: 2-(3,5-dichloropyridine-4-yl)-1-[(7-methoxy-1,3-benzodioxole-2-spirocyclopentane)-4-yl]ethan-1-one (Compound (VII-A))

n-butyl (7-methoxy-1,3-benzodioxole-2-spirocyclopentane)-4-carboxylate (119 g, 388 mmol) prepared in step 3 and 3,5-dichloro-4-picoline (100 g, 504 mmol) were dissolved in tetrahydrofuran (1.5 L). To the solution was added dropwise a 1.0 mol/L lithium bis(trimethylsilyl)amide in tetrahydrofuran (1,165 mL) at 0°C. The mixture was further stirred at 0°C for 1 hour. To the reaction mixture were added a saturated aqueous ammonium chloride (2.9 L) and water (480 ml), then the mixture was extracted. The organic layer was washed with a brine (1.4 L), and the solvent was evaporated under reduced pressure until a solid began to precipitate. Then, to the residue was added acetone (2.6 L), and the precipitated solid was dissolved at 50°C. The mixture was cooled to room temperature with stirring, and water (1.3 L) was slowly added dropwise. After being further stirring at room temperature for 30 minutes, a precipitated solid was filtered and washed with a mixed solvent of acetone and water (acetone:water = 1:1, 360 ml), followed by drying at 50°C for 6 hours to give the title compound (132 g, 86% yield; oveaall yield from 2,3,4-trimethoxybenzoic acid: 54%) as a white solid.
Melting point: 149° C
¹H NMR (300 MHz, CDCl₃) δ (ppm): 8.50 (s, 2H), 7.47 (d, J=9.1 Hz, 1H), 6.61 (d, J=9.1 Hz, 1H), 4.59 (s, 2H), 3.97 (s, 3H), 2.72-2.22 (m, 4H), 1.93-1.88 (m, 4H)
EI-MS: 393 (M⁺)
IR (KBr, cm⁻¹): 2968, 1684, 1636, 1504, 1441, 1393, 1335, 1286, 1180, 1101, 959, 787

### Industrial Applicability

According to the present invention, simple processes with a high yield for mass-producing a 1,3-benzodioxole-2-spirocycloalkane derivative which has inhibitory activity of PDE IV and is useful as therapeutic agents of, for example, an inflammatory or allergic disease such as bronchial asthma, allergic rhinitis, nephritis or the like; an autoimmune disease such as rheumatism, multiple sclerosis, Crohn's disease, psoriasis, systemic lupus erythematosus or the like; a disease of central nervous system such as depression, amnesia, dementia or the like; an organ disease with ischemia-reperfusion injury caused by, for example, cardiac failure, shock, cerebral ischemia injury or the like; insulin-resistant diabetes, wounds, AIDS or the like, and/or its intermediates are(is) provided.

## Claims

1. A process for preparing a 1,3-benzodioxole-2-spirocycloalkane derivative represented by formula (VII) 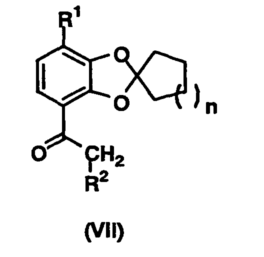 (wherein R¹ represents hydroxy, or substituted or unsubstituted lower alkoxy; R² represents substituted or unsubstituted aryl, or a substituted or unsubstituted aromatic heterocyclic group; and n represents an integer of from 1 to 6),
which comprises
treating a compound represented by formula (I) 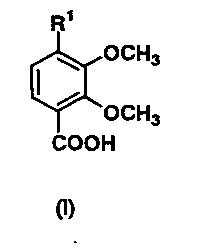 (wherein R¹ has the same meaning as defined above)
with hydrogen iodide to give a compound represented by formula (II) 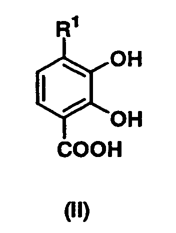 (wherein R¹ has the same meaning as defined above);
allowing the resulting compound represented by the above formula (II) to react with a compound represented by formula (III) 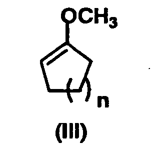 (wherein n has the same meaning as defined above)
to give a compound represented by formula (IV) 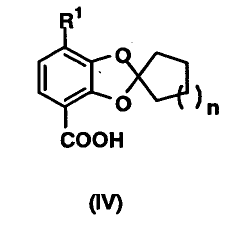 (wherein R¹ and n have the same meanings as defined above respectively);
converting the resulting compound represented by the above formula (IV) into a compound represented by formula (V) 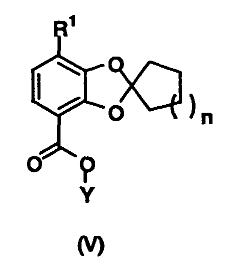 (wherein R¹ and n have the same meanings as defined above respectively; and Y represents lower alkyl, lower alkenyl, lower alkynyl, substituted or unsubstituted aralkyl, substituted or unsubstituted aryl, or a substituted or unsubstituted aromatic heterocyclic group);
adding a base to a mixture containing the resulting compound represented by the above formula (V) and a compound represented by formula (VI)
**R**^{**2**}**-CH**_{**3**} **(VI)**
(wherein R² has the same meaning as defined above);
and allowing the compound represented by the above formula (V) to react with the compound represented by the above formula (VI).

2. A process for preparing a 1,3-benzodioxole-2-spirocycloalkane derivative represented by formula (VII) 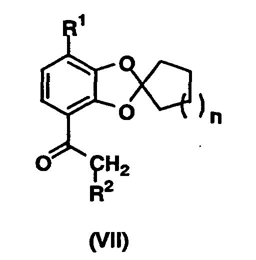 (wherein R¹, R² and n have the same meanings as defined above respectively)
which comprises
adding a base to a mixture containing a compound represented by formula (V) 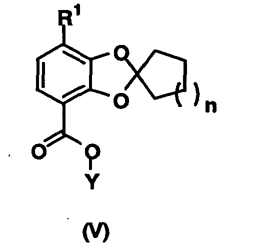 (wherein R¹, n and Y have the same meanings as defined above respectively)
and a compound represented by formula (VI)
**R**^{**2**}**-CH**_{**3**} **(VI)**
(wherein R² has the same meaning as defined above);
and allowing the compound represented by the above formula (V) to react with the compound represented by the above formula (VI).

3. The process for preparing a 1,3-benzodioxole-2-spirocycloalkane derivative according to Claim 1 or 2, wherein the base is lithium bis(trimethylsilyl)amide.

4. The process for preparing a 1,3-benzodioxole-2-spirocycloalkane derivative according to Claim 3, wherein the reaction temperature when the compound represented by formula (V) reacts with the compound represented by formula (VI) is between -10° C and 50°C.

5. The process for preparing a 1,3-benzodioxole-2-spirocycloalkane derivative according to any one of Claims 1 to 4, wherein Y is *n*-butyl.

6. A process for preparing a 1,3-benzodioxole-2-spirocycloalkane derivative represented by formula (VII) 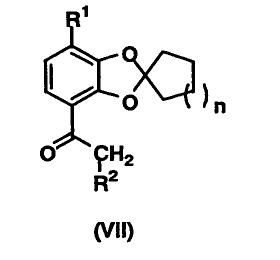 (wherein R¹, R² and n have the same meanings as defined above respectively)
which comprisis
allowing a compound represented by formula (II) 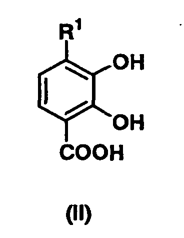 (wherein R¹ has the same meaning as defined above)
to react with a compound represented by formula (III) 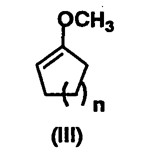 (wherein n represents an integer of from 1 to 6)
to give a compound represented by formula (IV) 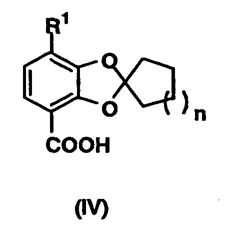 (wherein R¹ and n have the same meanings as defined above);
converting the resulting compound represented by the above formula (IV) into a compound represented by formula (V) 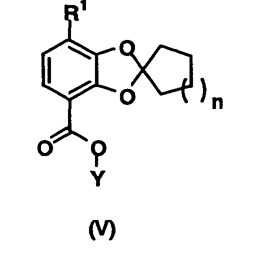 (wherein R¹, n and Y have the same meanings as defined above respectively);
adding a base to a mixture containing the resulting compound represented by the above formula (V) and a compound represented by formula (VI)
**R**^{**2**}**-CH**_{**3**} **(VI)**
(wherein R² has the same meaning as defined above);
and allowing the compound represented by the above formula (V) to react with the compound represented by the above formula (VI).

7. A process for preparing a compound represented by formula (IV) 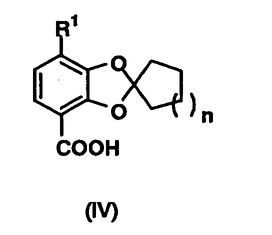 (wherein R¹ and n have the same meanings as defined above respectively)
which comprises
allowing a compound represented by formula (II) 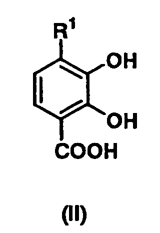 (wherein R¹ has the same meaning as defined above)
to react with a compound represented by formula (III) 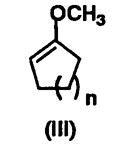 (wherein n has the same meaning as defined above).

8. A process for preparing a 1,3-benzodioxole-2-spirocycloalkane derivative represented by formula (VII) 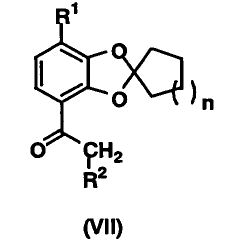 (wherein R¹, R² and n have the same meanings as defined above respectively)
which comprises
converting a compound represented by formula (IV) 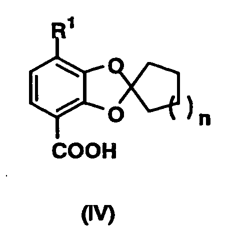 (wherein R¹ and n have the same meanings as defined above respectively)
into a compound represented by formula (V) 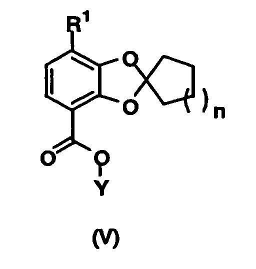 (wherein R¹, n and Y have the same meanings as defined above respectively);
adding a base to a mixture containing the resulting compound represented by the above formula (V) and a compound represented by formula (VI)
**R**^{**2**}**-CH**_{**3**} **(VI)**
(wherein R² has the same meaning as defined above);
and allowing the compound represented by the above formula (V) to react with the compound represented by the above formula (VI).

9. A process for preparing a compound represented by formula (II) 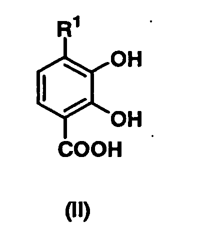 (wherein R¹ has the same meaning as defined above)
which comprises
treating a compound represented by formula (I) 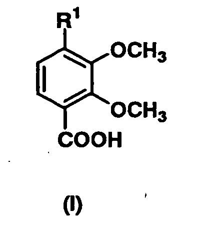 (wherein R¹ has the same meaning as defined above) with hydrogen iodide.

10. The process for preparing according to any one of Claims 1 to 6 and 8, wherein R² is a substituted or unsubstituted aromatic heterocyclic group.

11. The process for preparing according to any one of Claims 1 to 10, wherein R¹ is methoxy.
